# EUROPEAN PATENT SPECIFICATION

(11) **EP 1 144 113 B1**
(45) Date of publication and mention of the grant of the patent: **22.03.2006**
(21) Application number: 99947990.0
(22) Date of filing: 14.10.1999
(51) Int. Cl.: B01J 29/22

(54) **CATALYST FOR THE DISPROPORTIONATION/TRANSALKYLATION OF AROMATIC HYDROCARBONS AND METHOD FOR PREPARING THE SAME**
KATALYSATOR ZUR DISPROPORTIONIERUNG UND TRANSALKYLIERUNG VON AROMATISCHEN KOHLENWASSERSTOFFEN UND VERFAHREN ZU SEINER HERSTELLUNG
CATALYSEUR UTILISE POUR LA DISMUTATION/TRANSALKYLATION D'HYDROCARBURES AROMATIQUES ET PROCEDE DE PREPARATION DE CE DERNIER

(30) Priority: 24.12.1998 KR 9858628; 22.03.1999 KR 9909649
(43) Date of publication of application: 17.10.2001
(60) Divisional application: 05022191.0
(73) Proprietor: SK Corporation, Seoul 110-110 (KR)
(72) Inventor: OH, Seung, Hoon, 462-5, Yusung-ku, Taejon 305-390 (KR); LEE, Sang Il, Yusung-ku, Taejon 305-390 (KR); SEONG, Kyoung Hak, 462-5, Yusung-ku, Taejon 305-390 (KR); PARK, Sang Hoon, Seocho-ku, Seoul 137-030 (KR)
(74) Representative: Lins, Edgar
(86) International application number: PCT/KR1999/000619
(87) International publication number: WO 2000/038834

(56) References cited:
- EP-A1- 0 390 058
- EP-A1- 0 816 311
- DE-A- 19 641 149
- US-A- 4 939 110

## Description

### BACKGROUND OF THE INVENTION

### Field of the invention

The present invention relates, in general, to a catalyst for the disproportionation/transaklylation of aromatic hydrocarbons and, more particularly, to a catalyst which is useful to prepare mixed xylenes from a mixture of benzene, toluene and C9 or higher aromatic compounds through disproportionation/transalkylation. Also, the present invention is concerned with a method for preparing such a catalyst.

### Description of the Prior Art

Mixed xylenes, very important raw materials in the petrochemical industry, are composed of ethyl benzene and xylene isomers including meta-xylene, para-xylene and ortho-xylene. From mixed xylenes, thus, pure m-xylene, p-xylene and o-xylene, all important base fractions in the petrochemical industry, can be produced.

Owing to their similar boiling points, xylene isomers are very difficult to individually extract from the mixed xylenes by ordinary distillation processes. Usually, adsorptive separation, crystallization and/or isomerization are used to separate the individual isomers from the mixed xylenes.

To produce the mixed xylenes, the separation from the fractions rich in the mixed xylenes and the synthesis by reaction are commercially utilized. The former includes the separation from the reformed oils produced during the reforming of naphtha and the separation from the thermally cracked oils which are produced as by-products during thermal cracking. As for the latter, it can be exemplified by the disproportionation of toluene, the transalkylation of toluene/C9 aromatic hydrocarbons, and the alkylation of toluene with methanol.

The disproportionation/transalkylation catalysts which are used for commercial purposes are usually based on zeolites, such as mordenite and ZSM-5. For use, these zeolites are either molded or impregnated with catalytic metal components.

In many prior patents are found zeolite-based disproportionation/transalkylation catalysts.

U.S. Pat. No. 4,083,886 discloses a catalyst for transalkylating alkyl aromatic compounds such as toluene, which is prepared by molding mordenite with an inorganic oxide binder after being treated with aqueous ammonia. In contrast with the present invention, this catalyst does not employ a metal capable of hydrogenation, but performs transalkylation by zeolite itself. Toluene alone can be smoothly transalkylated by this catalyst. From a reaction containing C9 or higher aromatic hydrocarbons, however, a high yield of the mixed xylenes cannot be expected because the reaction is hard to dealkylate with the catalyst. In addition, the catalyst is deactivated faster as the proportion of C9 or higher aromatic hydrocarbons becomes larger.

U.S. pat. No. 5,030,787 discloses a transalkylating catalyst which is based on beta-zeolite whose acidity is weakened by a steam treatment. With the intent to reduce side products and to retard the deactivation of catalyst, the weakening of acidity is conducted. However, the weakening of the acidity without the introduction of a hydrogenating metal results in deteriorating the catalytic activity of the catalyst and thus, reducing the yield of the mixed xylenes.

U.S. Pat. No. 5,475,180 pertains to the disproportionation/transalkylation of toluene and high molecular weight aromatic hydrocarbons by use of a catalyst comprising nickel supported on mordenite. As in the present invention, the nickel functions to effectively dealkylate the aromatic hydrocarbons of large molecular weights and to suppress the deactivation of the catalyst. However, it is expected that this catalyst becomes deactivated faster during the catalytic reaction than does the catalyst employing activity-controlled platinum because the hydrogenation activity of nickel is far poorer than that of platinum. In addition, when sulfur compounds are introduced into the reaction, the hydrogenation activity of the nickel is greatly deteriorated owing to its strong association with the sulfur compounds. The feedstocks, which are fed into the commercial disproportionation/transalkylation process, usually undergo desulfurization in advance, but there is not completely excluded the possibility that sulfur compounds might flow into the disproportionation/transalkylation process owing to process accidents or operational errors. Upon the flowing in of sulfur compounds, the platinum catalyst has its platinum ingredient adsorbed with the sulfur compounds and thus, becomes deactivated temporarily. When the influx of sulfur compounds are stopped, however, the catalyst restores its catalytic activity because the sulfur compounds are desorbed from the platinum. In the case of nickel, the sulfur compounds, if adsorbed once, are very difficult to remove during reaction.

Another catalyst for the disproportionation/transalkylation of toluene and C9 aromatic hydrocarbons is found in U.S. Pat. No. 3,671,602 which discloses an alkali metal-deficient mordenite on which aluminum fluoride and a metal selected from the group consisting of Cu, Ag and Au or from the group consisting of W, Mo, Cr and As are supported, affirming that aluminum fluoride plays a role in restraining the production of coke so as to inhibit the deactivation of the catalyst. In this case the anti-deactivation effect cannot be efficiently conducted when the reaction contains C10 aromatic hydrocarbons or a high proportion of C9 aromatic hydrocarbons.

Also, the disproportionation/transalkylation of toluene and alkyl aromatic hydrocarbons is described in U.S. Pat. No.4,723,048 which discloses a catalyst comprising mordenite on which a metal of Group VIII, such as nickel or palladium, a metal of Group IB, such as Ag, and a metal of Group IVA, such as Sn, Pb or Ge, are supported. In this patent, the metal of Group VIII, such as nickel or palladium, serves as a hydrogenating metal whose activity is controlled by the metal of Group IVA, thereby improving the performance of the catalyst. The metals such as nickel and palladium are significantly poor in hydrogenation activity as compared with platinum, used in the present invention. When the reaction gets a high content of C9 or higher aromatic hydrocarbons, the catalyst is difficult to protect from deactivation. In addition, the presence of sulfur compounds in the reaction may make the hydrogenating activity of the catalyst drop to an unrecoverable state.

U.S. Pat. No. 5, 475, 179 discloses a catalyst for the disproportionation of toluene, which is based on Si-treated ZSM-5 type zeolite. It is also described that the treatment of ZSM-5 type zeolite with silicon makes the shape selectivity of the ZSM-5 type zeolite increase, so that the selectivity for the p-xylene of the mixed xylenes produced upon the disproportionation of toluene comes to reach about 90 wt% which is far higher than the thermodynamic equilibrium, 24 wt%. The use of ZSM-5 type zeolite alone, however, can be applied for toluene only, but cannot be applied for the disproportionation or transalkylation of C9 or higher aromatic hydrocarbons because of its structural limit.

DE 196 41 149 relates to a catalyst supported with noble metal(s) for the isomerisation of alkylaromatics containing platinum, rhenium and tin. Beside the isomerisation of C₈₋hydrocarbons, the isomerisation of C₉-hydrocarbons is described. However, no effect of the addition of rhenium and tin to a catalyst containing platinum on the isomerisation of Cg-hydrocarbons is demonstrated.

In addition, there are many examples of the disproportionation/transalkylation catalysts using mordenite, or beta type or ZSM-5 type zeolite as a base material, but nowhere is found a catalyst which uses platinum as a hydrogenating function and tin or lead as an activity controller so as to bring about a great improvement in the yield of mixed xylenes and in the deactivation of catalyst, as in the present invention.

### SUMMARY OF THE INVENTION

The intensive and thorough research on the development of a catalyst for the disproportionation/transalkylation of aromatic hydrocarbons, repeated by the present inventors aiming to solve the problems, resulted in the finding that platinum, together with tin or lead which plays a role as a controller of the high hydrogenating activity of platinum, is capable of producing mixed xylenes at high yields and exceptionally preventing the catalyst deactivation when being supported on a carrier consisting of mordenite or beta type, zeolite (in the following also referred to as beta) ZSM-5 type zeolite and an inorganic binder.

Therefore, it is an object of the present invention, based on this finding, to overcome the problems encountered in prior arts and to provide a catalyst for the disproportionation/transalkylation of benzene, toluene and C9 aromatic hydrocarbons, which allows mixed xylenes to be produced at remarkably high yields from benzene, toluene and C9 or higher aromatic compounds through disproportionation/transalkylation with a great reduction in aromatic loss and can maintain its catalytic activity for a long period of time without deactivation.

It is another object of the present invention to provide a method for preparing such a catalyst.

In accordance with a first aspect of the present invention, there is provided a catalyst for the disproportionation/transalkylation of aromatic hydrocarbons, which comprises: a carrier comprising 10 to 80 wt% of mordenite and/or beta type zeolite with a mole ratio of silica/alumina ranging from 10 to 200; 15 to 70 wt% of ZSM-5 type zeolite with a mole ratio of silica/alumina ranging from 30 to 500; and 5 to 90 wt% of at least one inorganic binder selected from the group consisting of gamma-alumina, silica, silica alumina, bentonite, kaolin, clinoptilolite, and montmorillonite, and metal components, which are supported on the carrier, consisting of platinum and either tin or lead, whereby mixed xylenes can be produced from benzene, toluene and C9 or higher aromatic hydrocarbons.

In accordance with a second aspect of the present invention, there is provided a method for preparing a catalyst for the disproportionation/transalkylation of various aromatic hydrocarbons, comprising the steps of a) forming a carrier which comprises 10 to 80 wt% of mordenite and/or beta type zeolite with a mole ratio of silica/alumina ranging from 10 to 200; 15 to 70 wt% of ZSM-5 type zeolite with a mole ratio of silica/alumina ranging from 30 to 500; and 5 to 90 wt% of at least one inorganic binder selected from the group consisting of gamma-alumina, silica, silica alumina, bentonite, kaolin, clinoptilolite, and montmorillonite; b) supporting 0.01 to 10.0 weight parts of tin or 0.01 to 7.0 weight parts of lead in 100 weight parts of the carrier on the carrier; and c) supporting 0.001 to 0.5 weight parts of platinum in 100 weight parts of the carrier on a tin or lead-supported carrier.

In accordance with a third aspect of the present invention, there is provided a method for preparing a catalyst for the disproportionation/transalkylation of various aromatic hydrocarbons, comprising the steps of: a) mixing 10 to 80 wt% of mordenite and/or beta type zeolite with a mole ratio of silica/alumina ranging from 10 to 200; 15 to 70 wt% of ZSM-5 type zeolite with a mole ratio of silica/alumina ranging from 30 to 500; 5 to 90 wt% of at least one inorganic binder selected from the group consisting of gamma-alumina, silica, silica alumina, bentonite, kaolin, clinoptilolite, and montmorillonite; 0.01 to 10.0 weight parts of tin or 0.01 to 7.0 weight parts of lead in 100 weight parts of the zeolite and the binder; and 0.001 to 0.5 weight parts of platinum in 100 weight parts of the zeolite and the binder; and b) molding the mixture.

In accordance with a fourth aspect of the present invention, there is provided a method for preparing a catalyst for the disproportionation/transalkylation of various aromatic hydrocarbons, comprising the steps of: a) supporting platinum on mordenite and/or beta type zeolite, ranging, in a mole ratio of silica/alumina, from 10 to 200, through impregnation or ion exchange; b) molding the platinum-supported mordenite and/or beta type zeolite with ZSM-5 type zeolite, ranging, in a mole ratio of silica/alumina, from 30 to 500 and at least one inorganic binder selected from the group consisting-of gamma-alumina, silica, silica alumina, bentonite, kaolin, clinoptilolite, and montmorillonite, into a certain form in which the mordenite and/or beta type zeolite, the ZSM-5 type zeolite, and the inorganic binder are present at an amount of 10 to 80 wt%, 15 to 70 wt%, and 5 to 90 wt%, respectively, said platinum amounting to 0.001 to 0.5 weight parts based on 100 weight parts of a carrier consisting of the zeolites and the binder; and c) supporting tin or lead in the molded form at an amount of 0.01 to 10.0 weight parts or 0.01 to 7.0 weight parts, respectively, based on 100 weight parts of the carrier.

In accordance with a fifth aspect of the present invention, there is provided a method for preparing a catalyst for the disproportionation/transalkylation of various aromatic hydrocarbons, comprising the steps of: a) supporting platinum on a mixture of at least one of mordenite and beta type zeolite, and ZSM-5 zeolite which ranges, in a mole ratio of silica/alumina, from 10 to 200 and from 30 to 500, respectively, through impregnation or ion exchange; b) molding the platinum-supported zeolite, together with at least one inorganic binder selected from the group consisting of gamma-alumina, silica, silica alumina, bentonite, kaolin, clinoptilolite, and montmorillonite, into a certain form in which the mordenite and/or beta type zeolite, the ZSM-5 type zeolite, and the inorganic binder are present at an amount of 10 to 80 wt%, 15 to 70 wt%, and 5 to 90 wt%, respectively, said platinum amounting to 0.001 to 0.5 weight parts based on 100 weight parts of a carrier consisting of the zeolites and the binder; and c) supporting tin or lead in the molded form at an amount of 0.01 to 10.0 weight parts or 0.01 to 7.0 weight parts, respectively, based on 100 weight parts of the carrier.

In accordance with a sixth aspect of the present invention, there is provided a method for preparing a catalyst for the disproportionation/transalkylation of various aromatic hydrocarbons, comprising the steps of: a) supporting tin or lead on mordenite and/or beta type zeolite, ranging, in a mole ratio of silica/alumina, from 10 to 200, through impregnation or ion exchange; b) molding the tin or lead-supported mordenite and/or beta type zeolite with ZSM-5 type zeolite, ranging, in a mole ratio of silica/alumina, from 30 to 500 and at least one inorganic binder selected from the group consisting of gamma-alumina, silica, silica alumina, bentonite, kaolin, clinoptilolite, and montmorillonite, into a certain form in which the mordenite and/or beta type zeolite, the ZSM-5 type zeolite, and the inorganic binder are present at an amount of 10 to 80 wt%, 15 to 70 wt%, and 5 to 90 wt%, respectively, said tin amounting to 0.01 to 10.0 weight parts or said lead amounting to 0.01 to 7.0 weight parts based on 100 weight parts of a carrier consisting of the zeolites and the binder; and c) supporting platinum in the molded form at an amount of 0.001 to 0.5 weight parts based on 100 weight parts of the carrier.

In accordance with a seventh aspect of the present invention, there is provided a method for preparing a catalyst for the disproportionation/transalkylation of various aromatic hydrocarbons, comprising the steps of: a) supporting tin or lead on a mixture of at least one of mordenite and beta type zeolite, and ZSM-5 zeolite which ranges, in a mole ratio of silica/alumina, from 10 to 200 and from 30 to 500, respectively, through impregnation or ion exchange; b) molding the tin or lead-supported zeolite, together with at least one inorganic binder selected from the group consisting of gamma-alumina, silica, silica alumina, bentonite, kaolin, clinoptilolite, and montmorillonite, into a certain form in which the mordenite and/or beta type zeolite, the ZSM-5 type zeolite, and the inorganic binder are present at an amount of 10 to 80 wt%, 15 to 70 wt%, and 5 to 90 wt%, respectively, said tin amounting to 0.01 to 10.0 weight parts or said lead amounting to 0.01 to 7.0 weight parts based on 100 weight parts of a carrier consisting of the zeolites and the binder; and c) supporting platinum in the molded form at an amount of 0.001 to 0.5 weight parts based on 100 weight parts of the carrier.

### BRIEF DESCRIPTION OF THE DRAWING

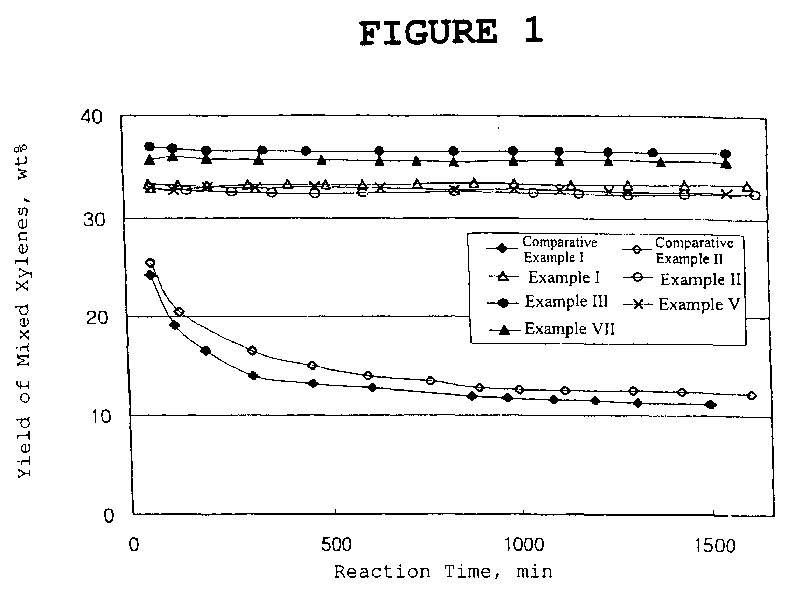

Fig. 1 is a graph in which the production yields of mixed xylenes are plotted with respect to reaction times for various catalysts.

### DETAILED DESCRIPTION OF THE INVENTION

The present invention pertains to a catalyst which is useful to produce mixed xylenes from benzene, toluene and C9 or higher aromatic hydrocarbons through disproportionation/transalkylation. On the catalyst, disproportionation between toluenes, transalkylation between toluene and C9 aromatic compounds, dealkylation of C9 or higher alkyl aromatic compounds, and transalkylation between benzene and C9 or higher aromatic hydrocarbons take place concurrently. This dealkylation is very important because toluene, needed for the disproportionation/transalkylation, is provided as a result of this reaction. Also, the transalkylation between benzene and C9 or higher aromatic hydrocarbons produces toluene and mixed xylenes.

In producing mixed xylenes from benzene, toluene and C9 or higher aromatic hydrocarbons, the olefins resulting from the dealkylation, such as ethylene and propylene, must be quickly hydrogenated; otherwise, the olefins are realkylated to the aromatic hydrocarbons, resulting in a decrease in the conversion rate of the C9 or higher aromatic hydrocarbons. Further, the olefins themselves are oligomerized to promote the production of cokes which give rise to deactivation in the catalyst. Herein, it should be noted that a metal capable of hydrogenation is contained, along with a zeolite base, such as mordenite, beta or ZSM-5, in the catalyst.

As a hydrogenating function, platinum is used in accordance with the present invention. To appropriately control the hydrogenating activity of platinum, tin or lead is employed. For better catalytic performance, this controlling metal is preferably used at an amount at least three times more than that of platinum.

Mordenite, beta and ZSM-5, which are useful zeolites in the present invention, are synthesized in sodium forms at first. The sodium forms are subjected to ion exchange with ammonium chloride or ammonium nitrate and these ammonium forms can be readily converted into hydrogen forms by calcination. In the present invention, an ammonium or hydrogen form of mordenite, beta or ZSM-5 is taken.

In accordance with the present invention, the mordenite or beta type zeolite ranges, in the mole ratio of silica/alumina, from 10 to 200. For instance, if the mole ratio of silica/alumina is below 10, the catalyst has too potent catalytic activity with an increasing of by-products and is deactivated too fast. On the other hand, if the zeolite has too high a mole ratio of silica/alumina, the resulting catalyst is so weak in catalytic activity that the production yield of mixed xylenes is poor.

As for ZSM-5, it has a mole ratio of silica/alumina from 30 to 500. As in the mordenite or beta, the mole ratio less than 30 provides the catalyst with too potent catalytic activity which causes an increase in by-products, forcing the catalyst to be deactivated too fast. On the other hand, if the mole ratio exceeds 500, the catalyst exerts a weak catalytic action on the aromatic hydrocarbons with a reduction in the production yield of mixed xylenes.

According to the present invention, the zeolites are combined with at least one inorganic binder. Examples of the inorganic binder usable in the present invention include gamma alumina, silica, silica alumina, bentonite, kaolin, clinoptilolite, and montmorillonite with preference to amorphous inorganic oxides of gamma alumina, silica and silica alumina and most preference to gamma alumina and silica.

When combining the inorganic binder with mordenite or beta type zeolite, and ZSM-5 to give a carrier, the mordenite or beta is preferably used at an amount of 10 to 80 wt%, ZSM-5 at an amount of 15 to 70 wt% and the inorganic binder at an amount of 5 to 90 wt%. For example, if the content of mordenite or beta is below 10 wt%, there is a deterioration in the production yield of mixed xylenes. On the other hand, a content greater than 80 wt% causes a problem of weakening the mechanical strength of the catalyst. ZSM-5, if used at an amount exceeding 70 wt%, has a bad effect on the production yield of mixed xylenes. As for the amount of the inorganic binder, less than 5 wt% causes a weakening in the mechanical strength of the catalyst while more than 90 wt% has a problem of lowering the production yield of mixed xylenes.

The mixture of mordenite or beta, ZSM-5 and the inorganic binder is extruded into a cylindrical shape in catalyst grains are 2 mm in diameter and 5 to 15 mm in length. Alternatively, the catalyst may be molded into a spherical shape. It should be noted that the catalyst may have any form. The molded carrier made of mordenite or beta, ZSM-5 and the inorganic binder preferably has the following physical properties: an apparent bulk density of 0.4 to 0.8 cc/g, an average pore diameter of 50 to 200 Å, a pore volume of 0.1 to 1 cc/g, and a specific surface area of 200 to 400 m²/g.

After the molding of mordenite or beta, ZSM-5 and the inorganic binder, the carrier is provided to make platinum/tin or platinum/lead supported thereon. Alternatively, the metal components may be supported on a mixture of mordenite or beta, and ZSM-5 before being molded along with the inorganic binder. The metal components may be introduced regardless of the time when the molding is conducted. Also, whether supporting the metal components before or after the molding, either of the two metals may be introduced first. Regardless of which of them is first introduced, there is a little difference in catalytic activity. Alternatively, the two metal components may be introduced concurrently. For example, the two metals are combined with the carrier mixture and then, molded together. Alternatively, either of them is combined with the carrier mixture before molding and then, the rest is supported on the resulting carrier to give a catalyst.

Platinum is preferably used at an amount of approximately 0.001 to 0.5 weight parts based on 100 weight parts of the carrier consisting of mordenite or beta, ZSM-5 and the inorganic binder. For example, if too little platinum is used, the resulting catalyst becomes of poor activity in terms of the dealkylation of alkyl aromatic compounds with reduction in the production yield of mixed xylenes while the catalyst is deactivated too fast. On the other hand, if platinum is used at an amount more than 0.5 weight parts based on 100 weight parts of the carrier, the resulting catalyst has too potent platinum activity, performing an active hydrocracking function to produce a significant amount of low molecular weight hydrocarbons (C1~C5).

The introduction of platinum to the catalyst structure may be achieved by ion exchange, impregnation or a physical mixing process. Upon the introduction by ion exchange, a solution of tetraamineplatinum chloride or tetraamine platinum nitrate in water may be used as a precursor for the platinum component. For the impregnation, a solution of hydrogen hexachloroplatinate or tetraamineplatinum chloride in water is used as a precursor for the platinum component. When a physical mixing process is taken to introduce platinum, any of the aqueous platinum solutions may be used.

In accordance with the present invention, tin, playing an important role in controlling the activity of the platinum, is preferably used at an amount of approximately 0.01 to 10.0 weight parts based on 100 weight parts of the carrier comprising mordenite or beta, ZSM-5 and the inorganic binder. For example, if the amount of tin exceeds 10.0 weight parts based on 100 weight parts of the carrier, the performance of platinum becomes so weak that there are caused problems of decreasing the production yield of mixed xylenes and promoting the deactivation of the catalyst. Lower than 0.01 weight parts of tin is insufficient to control the performance of platinum, resulting in high contents of low molecular weight hydrocarbons in the products. Tin is preferably introduced into the catalyst through impregnation or mixing. As a precursor for the tin component, stannous chloride, stannic chloride, tin acetate, or tin sulfate may be used.

In accordance with the present invention, lead may be used instead of tin. In the case of employing lead, the introduction of lead may be conducted in the same manner as in tin, with respect to amount, introduction route and precursor. It is preferred that the amount of lead is on the order of approximately 0.01 to 7.0 weight parts based on 100 weight parts of the carrier comprising mordenite or beta, ZSM-5 and the inorganic binder. In the catalyst, lead performs the same functions as tin and shows the same effects as those that tin does in dependence on its amount. Preferably, lead is introduced through impregnation or mixing. A precursor for the lead component may be selected from lead acetate, lead nitrate and lead sulfate.

After the introduction of the metal components related to hydrogenating functions is completed, the catalyst undergoes a drying process in air. It is preferable that the drying is conducted at a temperature of 60 to 200 °C for a period of 0.5 to 12 hours. Following the drying, a calcination process is applied for the catalyst. Preferably, the calcination process is carried out at a temperature of 300 to 650 °C for a period of 1 to 12 hours.

As previously mentioned, a pair of Pt/Sn or Pt/Pb, when being introduced onto the carrier comprising mordenite or beta, ZSM-5 and the inorganic binder, are indifferent to their introduction order. Instead, it is very important to make the metals associated suitably with each other. In order to provide better catalytic activity, particularly, platinum is in association with tin or lead or in the proximity of tin or lead sufficiently to have electrical and chemical influence on each other greater than does exist independently in the catalyst.

Platinum, if being in an independent state in the catalyst, exerts its high hydrogenation activity without control, effecting side reactions as above mentioned. However, when in association with or sufficiently near tin or lead, platinum is subject to tin or lead in its hydrogenation activity, so an optimal production yield of mixed xylenes can be obtained with sufficient retardation of the deactivation of the catalyst.

Being useful for the disproportionation of toluene and the transalkylation of toluene/C9 aromatic hydrocarbons, the catalyst according to the present invention is able to exert its effective performance when toluene or C9 or higher aromatic hydrocarbons (C9~C11 aromatic hydrocarbons) are fed, alone or in combination irrespective of their mole ratio of toluene/C9 or higher aromatic hydrocarbons and even when benzene is further fed.

As a result of subjecting benzene, toluene and C9 or higher aromatic hydrocarbons to disproportionation/transalkylation on the catalyst of the present invention, mixed xylenes were obtained at a yield of about 32 to 37 wt% with an aromatic loss amounting to as low as 2 wt%.

A better understanding of the present invention may be obtained in light of the following examples which are set forth to illustrate, but are not to be construed to limit the present invention.

### COMPARATIVE EXAMPLE I

A hydrogen form of mordenite with a mole ratio of silica/alumina of 90 was molded, together with gamma alumina as a binder, into a cylindrical shape 2 mm in diameter and 10 mm in length, so as to give a carrier in which the mordenite amounted to 50 wt%. After being dried at 150 °C for 10 hours, the carrier was calcined at 500 °C for 3 hours.

Without being further introduced with any metal, the carrier was tested for disproportionation/transalkylation. To this end, 2 g of the carrier were charged in a fixed-bed reactor and subjected to reduction at 900 °C for 2 hours in a hydrogen atmosphere. In the presence of the catalyst thus obtained, benzene, toluene and C9 or higher aromatic hydrocarbons were allowed to be subjected to disproportionation/transalkylation. The reaction results are given, along with reaction conditions, in Table 1 and Fig. 1.

### COMPARATIVE EXAMPLE II

A catalyst was prepared in the same manner as that of Comparative Example I, except for using a hydrogen form of beta zeolite with a mole ratio of silica/alumina of 25. The catalyst was tested for disproportionation/transalkylation as in Comparative Example I. The results are given as shown in Table 1.

### COMPARATIVE EXAMPLE III

A hydrogen form of mordenite with a mole ratio of silica/alumina of 90 and a hydrogen form of ZSM-5 with a mole ratio of silica/alumina of 80 were molded, together with gamma alumina as a binder, into a cylindrical shape 2 mm in diameter and 10 mm in length, so as to give a carrier in which the mordenite amounted to 50 wt%. After being dried at 150 °C for 10 hours, the carrier was calcined at 500 °C for 3 hours.

Without being further introduced with any metal, the carrier was tested for disproportionation/transalkylation under the same conditions as in Comparative Example I, but using a reactant mixture indicated in Table 1. The reaction results are given, along with reaction conditions, in Table 1 and Fig. 1.

### COMPARATIVE EXAMPLE IV

A catalyst was prepared in the same manner as that of Comparative Example I, except for using a hydrogen form of beta zeolite with a mole ratio of silica/alumina of 25, instead of mordenite. The catalyst was tested for disproportionation/transalkylation as in Comparative Example III. The reaction results are given as shown in Table 1.

### COMPARATIVE EXAMPLE V

After being prepared in the same manner as that of Comparative Example I, a carrier was treated with an aqueous H₂PtCl₆ solution such that 0.02 weight parts of platinum were impregnated in 100 weight parts of the carrier which was, then, dried at 150 °C for 10 hours, followed by calcination at 500 °C for 3 hours to give a catalyst. This was tested for the same disproportionation/transalkylation as in Comparative Example I, but using the reactant mixture indicated in Table 1. The reaction results are given in Table 1.

### COMPARATIVE EXAMPLE VI

A carrier was prepared in the same manner as in Comparative Example II and introduced with platinum under the same condition as in Comparative Example II to give a catalyst in the presence of which disproportionation/transalkylation was conducted as in Comparative Example V. The reaction results are given in Table 1.

### COMPARATIVE EXAMPLE VII

After being prepared in the same manner as that of Comparative Example III, a carrier was treated with an aqueous H₂PtCl₆ solution such that 0.02 weight parts of platinum were impregnated in 100 weight parts of the carrier which was, then, dried at 150 °C for 10 hours, followed by calcination at 500 °C for 3 hours to give a catalyst. This was tested for the same disproportionation/transalkylation as in Comparative Example I, but using the reactant mixture indicated in Table 1. The reaction condition and results are given in Table 1.

### COMPARATIVE EXAMPLE VIII

A carrier was prepared in the same manner as in Comparative Example IV and introduced with platinum under the same condition as in Comparative Example VII to give a catalyst in the presence of which disproportionation /transalkylation was conducted as in Comparative Example VII. The reaction results are given in Table 1.

**TABLE 1 Assay for Disproportionation/Transalkylation Performance**

| Nos. of C. Exmpl. | Aromatic Reactants (wt%) | | | Aromatic Products (wt%) | | | Yield of mixed xylenes (wt%) | Aromatic Loss (wt%) |
|---|---|---|---|---|---|---|---|---|
| | C6-C8 | C9 | C10 | C6-C8 | C9 | C10 | | |
| I | 51.2 | 45.1 | 3.5 | 58.4 | 36.8 | 4.5 | 11.8 | 0.3 |
| II | 51.2 | 45.1 | 3.5 | 58.8 | 36.6 | 4.5 | 12.1 | 0.2 |
| III | 22.1 | 74.1 | 2.6 | 59.6 | 30.8 | 7.1 | 22.8 | 0.2 |
| IV | 22.1 | 74.1 | 2.6 | 58.4 | 31.0 | 7.5 | 22.5 | 0.2 |
| V | 22.1 | 74.1 | 2.6 | 64.1 | 9.6 | 1.4 | 26.4 | 22.8 |
| VI | 22.1 | 74.1 | 2.6 | 63.5 | 10.0 | 1.5 | 25.5 | 21.0 |
| VII | 22.1 | 74.1 | 2.6 | 63.0 | 23.6 | 1.0 | 23.1 | 10.2 |
| VIII | 22.1 | 74.1 | 2.6 | 62.5 | 24.0 | 1.2 | 22.5 | 11.0 |

| | | | | | | | | |
|---|---|---|---|---|---|---|---|---|
| Reaction condition: Temp. 400 °C; Press. 27.9 kg/cm², WHSV=3hr⁻¹; H2/HC molar ratio=3 | | | | | | | | |

### Reference EXAMPLE I

A hydrogen form of mordenite with a mole ratio of silica/alumina of 90 was molded, together with gamma alumina as a binder, into a cylindrical shape 2 mm in diameter and 10 mm in length, so as to give a carrier in which the mordenite amounted to 50 wt%. After being dried at 150 °C for 10 hours, the carrier was calcined at 500 °C for 3 hours. Using an aqueous SnCl₂ solution, 0.5 weight parts of tin were impregnated in 100 weight parts of the carrier, which was then dried at 150 °C for 10 hours and calcined at 500 °C for 3 hours. This tin-impregnated carrier was treated with an aqueous H₂PtCl₆ solution such that 0.05 weight parts of platinum were impregnated in 100 weight parts of the mordenite and binder. The resulting carrier was subjected to drying at 150 °C for 10 hours and then to calcination at 500 °C for 3 hours to allow a catalyst.

For disproportionation/transalkylation testing, 2.0 g of the catalyst were charged in a fixed-bed reactor and subjected to reduction at 400 °C for 2 hours in a hydrogen atmosphere. In the presence of the catalyst thus activated, benzene, toluene and C9 or higher aromatic hydrocarbons were allowed to be subjected to disproportionation/transalkylation. The reaction results are given, along with reaction conditions, in Table 2 and Fig. 1.

### Reference EXAMPLE II

A catalyst was prepared in the same manner as that of Example I, except for using a hydrogen form of beta zeolite with a mole ratio of silica/alumina of 25. The catalyst was tested for disproportionation/transalkylation as in Example I, but using the reactant mixture indicated in Table 2. The results are given as shown in Table 2 and Fig. 1. As apparent from the data, this catalyst showed similar catalytic performance to that of the catalyst of Example I.

### EXAMPLE III

A hydrogen form of mordenite and a hydrogen form of ZSM-5 which had mole ratios of silica/alumina of 90 and 80, respectively, were molded, together with gamma alumina as a binder, into a cylindrical shape 2 mm in diameter and 10 mm in length, so as to give a carrier in which the mordenite and the ZSM-5 amounted to 40 wt% and 15 wt%, respectively. After being dried at 150 °C for 10 hours, the carrier was calcined at 500 °C for 3 hours. Using an aqueous SnCl₂ solution, 0.5 weight parts of tin were impregnated in 100 weight parts of the carrier, which was then dried at 150 °C for 10 hours and calcined at 500 °C for 3 hours. This tin-impregnated carrier was treated with an aqueous H₂PtCl₆ solution such that 0.05 weight parts of platinum were impregnated in 100 weight parts of the mordenite, ZSM-5 and binder. The resulting carrier was subjected to drying at 150 °C for 10 hours and then to calcination at 500 °C for 3 hours to allow a catalyst.

The catalyst was tested for disproportionation/transalkylation under the same conditions as in Example I, but using a reactant mixture indicated in Table 2. The reaction results are given in Table 2 and Fig. 1. The catalyst showed a similar reaction performance to and a little bit more improved production yield of mixed xylenes than the catalyst of Example I.

### EXAMPLE IV

A catalyst was prepared in the same manner as that of Example III, except for using a hydrogen form of beta zeolite with a mole ratio of silica/alumina of 25, instead of mordenite, and tested for disproportionation/transalkylation as in Example III. The reaction results are given as shown in Table 2. There was obtained a similar performance to that of Example III.

### Reference EXAMPLE V

While a hydrogen form of mordenite with a mole ratio of silica/alumina of 90 was molded, together with gamma-alumina as a binder, into a cylindrical shape 2 mm in diameter and 10 mm in length, an aqueous H₂PtCl₆ solution and an aqueous SnCl₂ solution were added so as to make platinum and tin be present at amounts of 0.04 and 0.4 weight parts, respectively, in 100 weight parts of the carrier consisting of the mordenite and the binder with the mordenite amounting to 50 wt%. Thereafter, the carrier was dried at 150 °C for 10 hours, followed by calcination at 500 °C for 3 hours to give a catalyst. This was tested for the same disproportionation/transalkylation as in Example I. The reaction conditions and results are given in Table 2 and Fig. 1.

### Reference EXAMPLE VI

A catalyst was prepared in the same manner as that of Example V, except for using a hydrogen form of beta zeolite with a mole ratio of silica/alumina of 25, instead of mordenite, and tested for disproportionation/transalkylation as in Example V. The reaction results are given as shown in Table 2.

### EXAMPLE VII

While hydrogen forms of mordenite and ZSM-5 which had mole ratios of silica/alumina of 90 and 80, respectively, were molded, together with gamma-alumina as a binder, into a cylindrical shape 2 mm in diameter and 10 mm in length, an aqueous H₂PtCl₆ solution and an aqueous SnCl₂ solution were added so as to make platinum and tin be present at amounts of 0.04 and 0.4 weight parts, respectively, in 100 weight parts of the carrier consisting of the mordenite, ZSM-5 and the binder with the mordenite and the ZSM-5 amounting to 40 wt% and 15 wt%, respectively. Thereafter, the carrier was dried at 150 °C for 10 hours, followed by calcination at 500 °C for 3 hours to allow a catalyst. This was tested for the same disproportionation/transalkylation as in Example I, but using the reactant mixture indicated in Table 2. The reaction conditions and results are given in Table 2 and Fig. 1.

### EXAMPLE VIII

A catalyst was prepared in the same manner as that of Example VII, except for using a hydrogen form of beta zeolite with a mole ratio of silica/alumina of 25, instead of mordenite and ZSM-5, and tested for disproportionation/transalkylation as in Example VII. The reaction results are given as shown in Table 2.

**TABLE 2 Assay for Disproportionation/Transalkylation Performance**

| | Nos. of Exmpl. | Aromatic Reactants (wt%) | | | Aromatic Products (wt%) | | | Yield of mixed xylenes (wt%) | Aromatic Loss (wt%) |
|---|---|---|---|---|---|---|---|---|---|
| | | C6-C8 | C9 | C10 | C6-C8 | C9 | C10 | | |
| Reference | I | 65.5 | 31.0 | 2.9 | 80.3 | 12.8 | 3.2 | 33. 3 | 1.3 |
| Reference | II | 65.5 | 31.0 | 2.9 | 79.8 | 12.6 | 3.0 | 32.5 | 1.4 |
| | III | 22.1 | 74.1 | 2.6 | 63.6 | 21.5 | 4.2 | 36.4 | 1.9 |
| | IV | 22.1 | 74.1 | 2.6 | 62.6 | 22.5 | 4.9 | 35.3 | 1.7 |
| Reference | V | 65.5 | 31.0 | 2.9 | 81.8 | 11.3 | 1.7 | 33.0 | 1.8 |
| Reference | VI | 65.5 | 31.0 | 2.9 | 79.5 | 12.6 | 2.9 | 32.1 | 2.3 |
| | VII | 22.1 | 74.1 | 2.6 | 63.1 | 21.6 | 4.5 | 35.7 | 1.8 |
| | VIII | 22.1 | 74.1 | 2.6 | 62.7 | 22.0 | 5.0 | 35.0 | 1.6 |

| | | | | | | | | | |
|---|---|---|---|---|---|---|---|---|---|
| Reaction condition: Temp. 400 °C; Press. 27.9 kg/cm², WHSV=3hr⁻¹; H2/HC molar ratio=3 | | | | | | | | | |

From the data of Table 2, it is recognized that mixed xylenes can be produced at even much higher yields using the catalysts according to the present invention than using the conventional catalysts. In addition, the catalyst of Examples I to VIII show significantly lowered aromatic loss as compared with the catalysts of Comparative Examples V to VIII.

With reference to Fig. 1, the production yields of mixed xylenes are traced with respect to reaction times for various catalysts. As shown in this graph, the catalysts according to the present invention maintain their activity constant through the reaction periods of time and thus, greatly improved in the catalyst deactivation compared with the conventional catalysts which are greatly deactivated within 500 min after the reaction.

### Reference EXAMPLE IX

The same procedure as that of Example I was repeated, except for using an aqueous Pb(NO₃)₂ solution instead of an aqueous SnCl₂ solution, to give a catalyst which contained lead at an amount of 0.6 weight parts based on 100 weight parts of the carrier. The catalyst was tested for disproportionation/transalkylation as in Example I, but using the reactant mixture indicated in Table 3. The reaction results are given in Table 3.

### Reference EXAMPLE X

The same procedure as that of Example II was repeated, except for using an aqueous Pb(NO₃)₂ solution instead of an aqueous SnCl₂ solution, to give a catalyst which contained lead at an amount of 0.6 weight parts based on 100 weight parts of the carrier. The catalyst was tested for disproportionation/transalkylation as in Example II, but using the reactant mixture indicated in Table 3. The reaction results are given in Table 3.

### EXAMPLE XI

The same procedure as that of Example III was repeated, except for using an aqueous Pb(NO₃)₂ solution instead of an aqueous SnCl₂ solution, to give a catalyst which contained lead at an amount of 0.6 weight parts based on 100 weight parts of the carrier. The catalyst was tested for disproportionation/transalkylation as in Example III, but using the reactant mixture indicated in Table 3. The reaction results are given in Table 3.

### EXAMPLE XII

The same procedure as that of Example IV was repeated, except for using an aqueous Pb(NO₃)₂ solution instead of an aqueous SnCl₂ solution, to give a catalyst which contained lead at an amount of 0.6 weight parts based on 100 weight parts of the carrier. The catalyst was tested for disproportionation/transalkylation as in Example IV, but using the reactant mixture indicated in Table 3. The reaction results are given in Table 3.

### Reference EXAMPLE XIII

The same procedure as that of Example V was repeated, except for using an aqueous Pb(NO₃)₂ solution, instead of an aqueous SnCl₂ solution, to give a catalyst which contained lead at an amount of 0.5 weight parts based on 100 weight parts of the carrier. The catalyst was tested for disproportionation/transalkylation as in Example V, but using the reactant mixture indicated in Table 3. The reaction results are given in Table 3.

### Reference EXAMPLE XIV

The same procedure as that of Example VI was repeated, except for using an aqueous Pb(NO₃)₂ solution instead of an aqueous SnCl₂ solution, to give a catalyst which contained lead at an amount of 0.5 weight parts based on 100 weight parts of the carrier. The catalyst was tested for disproportionation/transalkylation as in Example VI, but using the reactant mixture indicated in Table 3. The reaction results are given in Table 3.

### EXAMPLE XV

The same procedure as that of Example VII was repeated, except for using an aqueous Pb(NO₃)₂ solution instead of an aqueous SnCl₂ solution, to give a catalyst which contained lead at an amount of 0.5 weight parts based on 100 weight parts of the carrier. The catalyst was tested for disproportionation/transalkylation as in Example VII, but using the reactant mixture indicated in Table 3. The reaction results are given in Table 3.

### EXAMPLE XVI

The same procedure as that of Example VIII was repeated, except for using an aqueous Pb(NO₃)₂ solution instead of an aqueous SnCl₂ solution, to give a catalyst which contained lead at an amount of 0.5 weight parts based on 100 weight parts of the carrier. The catalyst was tested for disproportionation/transalkylation as in Example VIII, but using the reactant mixture indicated in Table 3. The reaction results are given in Table 3.

**TABLE 3 Assay for Disproportionation/Transalkylation Performance**

| | | | | | | | | | |
|---|---|---|---|---|---|---|---|---|---|
| | Nos. of Exmpl. | Aromatic Reactants (wt%) | | | Aromatic Products (wt%) | | | Yield of mixed xylenes (wt%) | Aromatic Loss (wt%) |
| | | C6-C8 | C9 | C10 | C6-C8 | C9 | C10 | | |
| Reference | IX | 21.1 | 63.9 | 13.3 | 61.3 | 18.7 | 8.2 | 33.5 | 1.8 |
| Reference | X | 21.1 | 63.9 | 13.3 | 60.1 | 19.3 | 8.9 | 33.0 | 1.5 |
| | XI | 22.1 | 74.1 | 2.6 | 64.0 | 21.1 | 4.3 | 36.6 | 2.0 |
| | XII | 22.1 | 74.1 | 2.6 | 64.3 | 20.9 | 4.1 | 36.8 | 2.2 |
| Reference | XIII | 22.1 | 74.1 | 2.6 | 61.7 | 18.5 | 7.9 | 33.7 | 1.9 |
| Reference | XIV | 22.1 | 74.1 | 2.6 | 61.3 | 18.8 | 8.1 | 33.0 | 1.9 |
| | XV | 22.1 | 74.1 | 2.6 | 62.0 | 22.3 | 5.0 | 35.3 | 1.4 |
| | XVI | 22.1 | 74.1 | 2.6 | 61.5 | 22.8 | 5.3 | 36.7 | 1.2 |

| | | | | | | | | | |
|---|---|---|---|---|---|---|---|---|---|
| Reaction condition: Temp. 400 °C; Press. 27.9 kg/cm², WHSV=3hr⁻¹; H2/HC molar ratio=3 | | | | | | | | | |

### Reference EXAMPLE XVII

After being impregnated with platinum by use of an aqueous H₂PtCl₆ solution, a hydrogen form of mordenite with a mole ratio of silica/alumina of 90 was molded, together with gamma alumina as a binder, into a cylindrical shape 2 mm in diameter and 10 mm in length, so as to give a platinum-impregnated carrier in which the mordenite amounted to 50 wt% based on the total weight of the carrier consisting of the mordenite and the gamma alumina binder and the platinum amounted to 0.04 weight parts based on 100 weight parts of the carrier. The platinum-impregnated carrier was dried at 150 °C for 10 hours and then, calcined at 500 °C for 3 hours. Using an aqueous SnCl₂ solution, 0.4 weight parts of tin were impregnated in 100 weight parts of the carrier, which was then dried at 150 °C for 10 hours and calcined at 500 °C for 3 hours to allow a catalyst.

The catalyst was tested for disproportionation/transalkylation under the same conditions as in Example I, but using a reactant mixture indicated in Table 4. The reaction results are given in Table 4.

### Reference EXAMPLE XVIII

A catalyst was prepared in the same manner as that of Example XVII, except for using a hydrogen form of beta zeolite with a mole ratio of silica/alumina of 25. The catalyst was tested for disproportionation/transalkylation as in Example XVII. The results are given as shown in Table 4 .

### Reference EXAMPLE XIX

After being impregnated with platinum by use of an aqueous H₂PtCl₆ solution, a hydrogen form of mordenite with a mole ratio of silica/alumina of 90 was mixed with ZSM-5 with a mole ratio of silica/alumina of 80, after which this mixture was molded, together with gamma alumina as a binder, into a cylindrical shape 2 mm in diameter and 10 mm in length, so as to give a platinum-impregnated carrier in which the mordenite and the ZSM-5 amounted to 40 wt% and 15 wt%, respectively, based on the total weight of the carrier consisting of the mordenite, the ZSM-5 and the gamma alumina and the platinum amounted to 0.04 weight parts based on 100 weight parts of the carrier. The platinum-impregnated carrier was dried at 150 °C for 10 hours and then, calcined at 500 °C for 3 hours. Using an aqueous SnCl₂ solution, 0.4 weight parts of tin were impregnated in 100 weight parts of the carrier, which was then dried at 150 °C for 10 hours and calcined at 500 °C for 3 hours to allow a catalyst.

The catalyst was tested for disproportionation/transalkylation under the same conditions as in Example I, but using a reactant mixture indicated in Table 4. The reaction results are given in Table 4.

### EXAMPLE XX

A catalyst was prepared in the same manner as that of Example XIX, except for using a hydrogen form of beta zeolite with a mole ratio of silica/alumina of 25. The catalyst was tested for disproportionation/transalkylation as in Example XIX, but using the reactant mixture indicated in Table 4. The results are given as shown in Table 4.

### EXAMPLE XXI

A mixture of a hydrogen form of mordenite and a hydrogen form of ZSM-5 which had mole ratios of silica/alumina of 90 and 80, respectively, was treated with an aqueous H₂PtCl₆ solution such that platinum was impregnated at an amount of 0.075 wt% in the mordenite and at an amount of 0.067 wt% in the ZSM-5. Thereafter, the resulting mixture was molded, together with gamma alumina as a binder, into a cylindrical 2 mm in diameter and 10 mm in length, so as to give a platinum-impregnated carrier in which the mordenite and the ZSM-5 amounted to 40 wt% and 15 wt%, respectively, based on the total weight of the carrier consisting of the mordenite, the ZSM-5 and the gamma alumina. The platinum-impregnated carrier was dried at 150 °C for 10 hours and then, calcined at 500 °C for 3 hours. Using an aqueous SnCl₂ solution, 0.4 weight parts of tin were impregnated in 100 weight parts of the carrier, which was then dried at 150 °C for 10 hours and calcined at 500 °C for 3 hours to allow a catalyst.

The catalyst was tested for disproportionation/transalkylation under the same conditions as in Example I, but using a reactant mixture indicated in Table 4. The reaction results are given in Table 4.

### EXAMPLE XXII

A catalyst was prepared in the same manner as that of Example XXI, except for using a hydrogen form of beta zeolite with a mole ratio of silica/alumina of 25. The catalyst was tested for disproportionation/transalkylation as in Example XXI. The results are given as shown in Table 4 .

### Reference EXAMPLE XXIII

A catalyst was prepared in the same manner as that of Example XVII, except that an aqueous Pb(NO₃)₂ solution, instead of an aqueous SnCl₂ solution, was used to impregnate of 0.5 weight parts of lead in 100 weight parts of the carrier. The catalyst was tested for disproportionation/transalkylation as in Example XVII. The results are given as shown in Table 4.

### EXAMPLE XXIV

A catalyst was prepared in the same manner as that of Example XXI, except that an aqueous Pb(NO₃)₂ solution, instead of an aqueous SnCl₂ solution, was used to impregnate 0.5 weight parts of lead in 100 weight parts of the carrier. T h e catalyst was tested for disproportionation/transalkylation as in Example XXI. The results are given as shown in Table 4.

**TABLE 4 Assay for Disproportionation/Transalkylation Performance**

| | Nos. of Exmpl. | Aromatic Reactants (wt%) | | | Aromatic Products (wt%) | | | Yield of mixed xylenes (wt%) | Aromatic Loss (wt%) |
|---|---|---|---|---|---|---|---|---|---|
| | | C6-C8 | C9 | C10 | C6-C8 | C9 | C10 | | |
| Reference | XVII | 21.1 | 63.9 | 13.3 | 61.0 | 18.1 | 7.8 | 33.1 | 1.6 |
| Reference | XVIII | 21.1 | 63.9 | 13.3 | 60.2 | 18.0 | 7.6 | 32.3 | 1.7 |
| | XIX | 22.1 | 74.1 | 2.6 | 63.0 | 20.5 | 4.6 | 35.5 | 1.7 |
| | XX | 22.1 | 74.1 | 2.6 | 62.5 | 20.7 | 4.9 | 34.9 | 1.3 |
| | XXI | 22.1 | 74.1 | 2.6 | 61.5 | 23.0 | 5.6 | 35.1 | 1.0 |
| | XXII | 22.1 | 74.1 | 2.6 | 62.9 | 22.0 | 5.1 | 35.7 | 1.2 |
| Reference | XXIII | 22.1 | 63.9 | 13.3 | 60.5 | 18.0 | 7.2 | 32.7 | 1.8 |
| | XXIV | 22.1 | 74.1 | 2.6 | 62.7 | 21.6 | 4.4 | 35.7 | 1.8 |

| | | | | | | | | | |
|---|---|---|---|---|---|---|---|---|---|
| Reaction condition: Temp. 400 °C; Press. 27.9 kg/cm², WHSV=3hr⁻¹, H2/HC molar ratio=3 | | | | | | | | | |

### Reference EXAMPLE XXV

After being impregnated with tin by use of an aqueous SnCl₂ solution, a hydrogen form of mordenite with a mole ratio of silica/alumina of 90 was molded, together with gamma alumina as a binder, into a cylindrical shape 2 mm in diameter and 10 mm in length, so as to give a tin-impregnated carrier in which the mordenite amounted to 50 wt% based on the total weight of the carrier consisting of the mordenite and the gamma alumina binder and the tin amounted to 0.4 weight parts based on 100 weight parts of the carrier. The tin-impregnated carrier was dried at 150 °C for 10 hours and then, calcined at 500 °C for 3 hours. The resulting tin-contained carrier was treated with an aqueous H₂PtCl₆ solution such that 0.04 weight parts of platinum were impregnated in 100 weight parts of the carrier consisting of the mordenite and the binder. Thereafter, the resulting carrier was dried at 150 °C for 10 hours and calcined at 500 °C for 3 hours to allow a catalyst. This was tested for the same disproportionation/ transalkylation as in Example I, but using the reactant mixture indicated in Table 5. The reaction conditions and results are given in Table 5.

### Reference EXAMPLE XXVI

A catalyst was prepared in the same manner as that of Example XXV, except for using a hydrogen form of beta zeolite with a mole ratio of silica/alumina of 25. The catalyst was tested for disproportionation/transalkylation as in Example XXV. The results are given as shown in Table 5.

### EXAMPLE XXVII

After being impregnated with tin by use of an aqueous SnCl₂, solution, a hydrogen form of mordenite with a mole ratio of silica/alumina of 90 was mixed with ZSM-5 with a mole ratio of silica/alumina of 80, after which this mixture was molded, together with gamma alumina as a binder, into a cylindrical shape 2 mm in diameter and 10 mm in length, so as to give a tin-impregnated carrier in which the mordenite and the ZSM-5 amounted to 40 wt% and 15 wt%, respectively, based on the total weight of the carrier consisting of the mordenite, the ZSM-5 and the gamma alumina and the tin amounted to 0.4 weight parts based on 100 weight parts of the carrier. The tin-impregnated carrier was dried at 150 °C for 10 hours and then, calcined at 500 °C for 3 hours.

The resulting tin-contained carrier was treated with an aqueous H₂PtCl₆ solution such that 0.04 weight parts of platinum were impregnated in 100 weight parts of the carrier consisting of the mordenite, the ZSM-5 and the binder. Thereafter, the resulting carrier was dried at 150 °C for 10 hours and calcined at 500 °C for 3 hours to allow a catalyst. This was tested for the same disproportionation/transalkylation as in Example I, but using the reactant mixture indicated in Table 5. The reaction conditions and results are given in Table 5.

### EXAMPLE XXVIII

A catalyst was prepared in the same manner as that of Example XXVII, except for using a hydrogen form of beta zeolite with a mole ratio of silica/alumina of 25. The catalyst was tested for disproportionation/transalkylation as in Example XXVII. The results are given as shown in Table 5.

### Reference EXAMPLE XXIX

A catalyst was prepared in the same manner as that of Example XXV, except that an aqueous Pb(NO₃)₂ solution, instead of an aqueous SnCl₂ solution, was used to impregnate 0.5 weight parts of lead in 100 weight parts of the carrier. The catalyst was tested for disproportionation/transalkylation as in Example XXV. The results are given as s.hown in Table 5.

### EXAMPLE XXX

A catalyst was prepared in the same manner as that of Example XXVII, except that an aqueous Pb(NO₃)₂ solution, instead of an aqueous SnCl₂. solution, was used to impregnate 0.5 weight parts of lead in 100 weight parts of the carrier. The catalyst was tested for disproportionation/transalkylation as in Example XXVII. The results are given as shown in Table 5.

**TABLE 5 Assay for Disproportionation/Transalkylation Performance**

| | Nos. of Exmpl. | Aromatic Reactants (wt%) | | | Aromatic Products (wt%) | | | Yield of mixed xylenes (wt%) | Aromatic Loss (wt%) |
|---|---|---|---|---|---|---|---|---|---|
| | | C6-C8 | C9 | C10 | C6-C8 | C9 | C10 | | |
| Reference . | XXV | 21.1 | 63.9 | 13.3 | 61.0 | 18.3 | 8.1 | 33.3 | 1.7 |
| Reference | XXVI | 21.1 | 63.9 | 13.3 | 60.1 | 19.0 | 8.5 | 32.2 | 1.2 |
| | XXVII | 22.1 | 74.1 | 2.6 | 61.7 | 23.6 | 6.1 | 35.0 | 0.8 |
| | XXVIII | 22.1 | 74.1 | 2.6 | 62.0 | 23.3 | 5.7 | 35.2 | 1.2 |
| Reference | XXIX | 21.1 | 63.9 | 13.3 | 61.9 | 18.3 | 7.7 | 32.9 | 1.9 |
| | XXX | 22.1 | 74.1 | 2.6 | 62.3 | 22.4 | 4.7 | 35.6 | 1.4 |

| | | | | | | | | | |
|---|---|---|---|---|---|---|---|---|---|
| Reaction condition: Temp. 400 °C; Press. 27.9 kg/cm², WHSV=3hr⁻¹; H2/HC molar ratio=3 | | | | | | | | | |

### Reference EXAMPLE XXXI

A catalyst was prepared in a similar manner to that of Example I, except that a mixture of a hydrogen form of mordenite and a hydrogen form of beta zeolite which had mole ratios of silica/alumina of 90 and 25, respectively, was molded, together with gamma alumina as a binder, into a cylindrical shape 2 mm in diameter and 10 mm in length, so as to allow a carrier in which the mordenite, the beta zeolite and the gamma alumina amounted to 30 wt%, 25 wt% and 45 wt%, respectively. The catalyst was tested for disproportionation/transalkylation as in Example I. The results are given as shown in Table 6.

### EXAMPLE XXXII

A catalyst was prepared in a similar manner to that of Example I, except that a mixture of a hydrogen form of mordenite, a hydrogen form of beta zeolite and a hydrogen form of ZSM-5 which had mole ratios of silica/alumina of 90, 25 and 80, respectively, was molded, together with gamma alumina as a binder, into a cylindrical shape 2 mm in diameter and 10 mm in length, so as to allow a carrier in which the mordenite, the beta zeolite, the ZSM-5 and the gamma alumina amounted to 20 wt%, 20 wt%, 15 wt% and 45 wt%, respectively. The catalyst was tested for disproportionation/transalkylation as in Example I, but using the reactant mixture indicated in Table 6. The results are given as shown in Table 6.

### Reference EXAMPLE XXXIII

A catalyst was prepared in the same manner as that of Example XXXI, except that an aqueous Pb(NO₃), solution, instead of an aqueous SnCl₂ solution, was used to impregnate 0.5 weight parts of lead in 100 weight parts of the carrier. The catalyst was tested for disproportionation/transalkylation as in Example XXXI. The results are given as shown in Table 6.

### EXAMPLE XXXIV

A catalyst was prepared in the same manner as that of Example XXXII, except that an aqueous Pb(NO₃)₂, solution, instead of an aqueous SnCl₂, solution, was used to impregnate 0.5 weight parts of lead in 100 weight parts of the carrier. The catalyst was tested for disproportionation/transalkylation as in Example XXXII. The results are given as shown in Table 6.

**TABLE 6 Assay for Disproportionation/Transalkylation Performance**

| | Nos. of Exmpl. | Aromatic Reactants (wt%). | | | Aromatic Products (wt%) | | | Yield of mixed xylenes (wt%) | Aromatic Loss (wt%) |
|---|---|---|---|---|---|---|---|---|---|
| | | C6-C6 | C9 | C10 | C6-CB | C9 | C10 | | |
| Reference | XXXI | 65.5 | 31 | 2.9 | 79.5 | 13.0 | 3.7 | 32.7 | 1.2 |
| | XXXII | 22.1 | 74.1 | 2.6 | 63.0 | 22.0 | 4.8 | 36.0 | 1.5 |
| Reference | XXXIII | 65.5 | 31 | 2.9 | 78.9 | 13.4 | 4.0 | 32.5 | 1.0 |
| | XXXIV | 22.1 | 74.1 | 2.6 | 64.5 | 21.2 | 4.1 | 36.7 | 1.9 |

| | | | | | | | | | |
|---|---|---|---|---|---|---|---|---|---|
| Reaction condition: Temp. 400 °C; Press. 27.9 kg/cm, WHSV=3hr⁻¹, H2/HC molar ratio =3 | | | | | | | | | |

As described hereinbefore, the catalysts according to the present invention enable mixed xylenes to be produced at remarkably high yields from benzene, toluene and C9 or higher aromatic compounds through disproportionation/transalkylation with a great reduction in aromatic loss. In addition, the catalysts can maintain their catalytic activity for a long period of time without deactivation. Therefore, they can be usefully applied for industrial purposes.

The present invention has been described in an illustrative manner, and it is to be understood the terminology used is intended to be in the nature of description rather than of limitation. Many modifications and variations of the present invention are possible in light of the above teachings. Therefore, it is to be understood that within the scope of the appended claims, the invention may be practiced otherwise than as specifically described.

## Claims

1. A catalyst comprising:
(1) a carrier comprising:
(a) 10 to 80 wt% of at least one of
(i) mordenite and
(ii) beta type zeolite,
said mordenite and said beta type zeolite having a mole ratio of silica to alumina of from 10 to 200,
(b) 15 to 70 wt% of ZSM-5 type zeolite having a mole ratio of silica to alumina of from 30 to 500; and
(c) 5 to 90 wt% of at least one inorganic binder selected from the group consisting of gamma-alumina, silica, silica-alumina, bentonite, kaolin, clinoptilolite and montmorillonite; and
(2) a metal component, supported on said carrier, said metal component consisting of:
(a) platinum, and
(b) either tin or lead.

2. The catalyst of claim 1, wherein the platinum is present in the carrier in an amount of from 0.001 to 0.5 weight parts based on 100 weight parts of the carrier.

3. The catalyst of claims 1 to 2, wherein the tin is present in the carrier in an amount of from 0.01 to 10.0 weight parts based on 100 weight parts of the carrier.

4. The catalyst of claims 1 to 3, wherein the lead is present in the carrier in an amount of from 0.01 to 7.0 weight parts based on 100 weight parts of the carrier.

5. The catalyst of claims 1 to 4, wherein the metal component consists of platinum and tin.

6. The catalyst of claims 1 to 2 or claim 4, wherein the metal component consists of platinum and lead.

7. A process for the preparation of a catalyst according to any one of claims 1 to 6, comprising the steps of:
(1) forming a carrier which comprises
(a) 10 to 80 wt% of at least one of
(i) mordenite and
(ii) beta type zeolite;
said mordenite and said beta type zeolite having a mole ratio of silica to alumina from 10 to 200;
(b) 15 to 70 wt% of ZSM-5 type zeolite having a mole ratio of silica to alumina of from 30 to 500; and
(c) 5 to 90 wt% of at least one inorganic binder selected from the group consisting of gamma-alumina, silica, silica alumina, bentonite, kaolin, clinoptilolite and montmorillonite;
(2) supporting 0.01 to 10.0 weight parts of tin or 0.01 to 7.0 weight parts of lead in 100 weight parts of said carrier; and
(3) supporting 0.001 to 0.5 weight parts of platinum on said tin- or lead-supported carrier, based on 100 weight parts of said carrier, to yield said catalyst.

8. A process for the preparation of a catalyst according to any one of claims 1 to 6, comprising the steps of:
(1) mixing
(a) 10 to 80 wt% of at least one of:
(i) mordenite and
(ii) beta type zeolite;
said mordenite and said beta type zeolite having a mole ratio of silica to alumina of from 10 to 200;
(b) 15 to 70 wt% of ZSM-5 type zeolite having a mole ratio of silica to alumina of from 30 to 500;
(c) 5 to 90 wt% of at least one inorganic binder selected from the group consisting of gamma-alumina, silica, silica alumina, bentonite, kaolin, clinoptilolite and montmorillonite;
(d) 0.01 to 10.0 weight parts of tin or 0.01 to 7.0 weight parts of lead in 100 weight parts of the carrier; and
(e) 0.001 to 0.5 weight parts of platinum in 100 weight parts of the carrier; and
(2) molding the mixture to yield said catalyst.

9. A process for the preparation of a catalyst according to any one of claims 1 to 6, comprising the steps of:
(1) supporting platinum on at least one of:
(i) mordenite and
(ii) beta type zeolite;
said mordenite and said beta type zeolite having a mole ratio of silica to alumina from 10 to 200;
through impregnation or ion exchange;
(2) molding said platinum, supported on at least one of (i) mordenite and (ii) beta type zeolite, with ZSM-5 type zeolite having a mole ratio of silica to alumina of from 30 to 500, and at least one inorganic binder selected from the group consisting of gamma alumina, silica, silica alumina, bentonite, kaolin, clinoptilolite and montmorillonite, into a certain form;
wherein;
the at least one of:
(i) mordenite and
(ii) beta type zeolite;
the ZSM-5 type zeolite, and
the inorganic binder;
are present in an amount of 10 to 80 wt%, 15 to 70 wt%, and 5 to 90 wt%, respectively and platinum is present in an amount of 0.001 to 0.5 weight parts based on 100 weight parts of the carrier consisting of zeolites and binder, and
(3) supporting tin or lead in said molded form at an amount of 0.01 to 10.0 weight parts or 0.01 to 7.0 weight parts, respectively, based on 100 weight parts of said carrier, to yield said catalyst.

10. A process for the preparation of a catalyst according to any one of claims 1 to 6, comprising the steps of:
(1) supporting platinum on a mixture of:
(a) at least one of
(i) mordenite and
(ii) beta type zeolite; said mordenite and said beta type zeolite having a mole ratio of silica to alumina from 10 to 200; and
(b) ZSM-5 zeolite in a mole ratio of silica to alumina of from 30 to 500; through impregnation or ion exchange;
(2) molding the platinum-supported zeolite together with at least one inorganic binder selected from the group consisting of gamma-alumina, silica, silica alumina, bentonite, kaolin, clinoptilolite and montmorillonite, into a certain form wherein the mordenite and/or beta type zeolite, the ZSM-5 zeolite and the binder are present in an amount of 10 to 80 wt%, 15 to 70 wt% and 5 to 90 wt%, respectively, and the platinum is present in an amount of 0.001 to 5 weight parts based on 100 weight parts of the carrier consisting of the zeolites and the binder; and
(3) supporting tin or lead in said molded form at an amount of 0.01 to 10.0 weight parts or 0.01 to 7.0 weight parts, respectively, based on 100 weight parts of the carrier, to yield said catalyst.

11. A process for the preparation of a catalyst according to any one of claims 1 to 6, comprising the steps of:
(1) supporting tin or lead on at least one of
(i) mordenite and
(ii) beta type zeolite;
said mordenite and said beta type zeolite having a mole ratio of silica to alumina from 10 to 200;
through impregnation or ion exchange
(2) molding the at least one of
(i) tin- or lead-supported mordenite; and
(ii) tin- or lead-supported beta type zeolite;
with
ZSM-5 zeolite having a mole ratio of silica to alumina from 30 to 500; and
at least one inorganic binder selected from the group consisting of gamma-alumina, silica, silica alumina, bentonite, kaolin, clinoptilolite and montmorillonite, into a certain form;
wherein:
the at least one of
(i) mordenite; and
(ii) beta type zeolite;
the ZSM-5 type zeolite, and
the inorganic binder
are present in an amount of 10 to 80 wt%, 15 to 70 wt%, and 5 to 90 wt%, respectively and said tin is present in an amount of 0.01 to 10 weight parts or said lead is present in an amount of 0.01 to 7.0 weight parts based on 100 weight parts of said carrier; and
(3) supporting 0.001 to 0.5 weight parts of platinum, based on 100 weight parts of the carrier, in said molded form, to yield said catalyst.

12. A process for the preparation of a catalyst according to any one of claims 1 to 6, comprising the steps of:
(1) supporting tin or lead on a mixture of
(a) one of
(i) mordenite and
(ii) beta type zeolite;
said mordenite and said beta type zeolite having a mole ratio of silica to alumina of from 10 to 200; and
(b) ZSM-5 zeolite having a mole ratio of silica to alumina of from 30 to 500,
through impregnation or ion exchange;
(2) molding the tin- or lead-supported zeolite together with at least on inorganic binder selected from the group consisting of gamma-alumina, silica, silica alumina, bentonite, kaolin, clinoptilolite and montmorrilonite, into a certain form wherein the mordenite and/or beta type zeolite, the ZSM-5 zeolite and the binder are present in an amount of 10 to 80 wt%, 15 to 70wt% and 5 to 90 wt%, respectively, and the tin is present in an amount of 0.01 to 10 weight parts or the lead is present in an amount of 0.01 to 7 weight parts based on 100 weight parts of said zeolites and binder; and
(3) supporting platinum in said molded form at an amount of 0.001 to 0.5 weight parts, based on 100 weight parts of the carrier, to yield said catalyst.

13. A process for producing mixed xylenes from aromatic hydrocarbons by dealkylation, disproportionation or transalkylation using the catalyst according to any of claims 1 to 6.

## Patentansprüche

1. Katalysator umfassend
(1) einen Träger umfassend:
(a) 10 bis 80 Gew.-% von mindestens einem von
(i) Mordenit und
(ii) Zeolith vom beta-Typ,
das Mordenit und das Zeolith vom beta-Typ haben ein Molverhältnis von Silica zu Alumina von 10 bis 200,
(b) 15 bis 70 Gew.-% Zeolith vom ZSM-5 Typ mit einem Molverhältnis von Silica zu Alumina von 30 bis 500; und
(c) 5 bis 90 Gew.-% mindestens eines organischen Bindemittels, ausgewählt aus der Gruppe bestehend aus Gamma-Alumina, Silica, Silica-Alumina, Bentonit, Kaolin, Clinoptilolith sowie Montmorillonit; und
(2) einer Metallkomponente geträgert auf diesen Träger, diese Metallkomponente besteht aus:
(a) Platin, und
(b) entweder Zinn oder Blei.

2. Katalysator gemäß Anspruch 1, wobei das Platin in dem Träger in einer Menge von 0,001 bis 0,5 Gewichtsteilen bezogen auf 100 Gewichtsteile des Trägers vorhanden ist.

3. Katalysator gemäß Ansprüchen 1 bis 2, wobei das Zinn in dem Träger in einer Menge von 0,01 bis 10,0 Gewichtsteilen bezogen auf 100 Gewichtsteile des Trägers vorhanden ist.

4. Katalysator gemäß Ansprüchen 1 bis 3, wobei das Blei in dem Träger in einer Menge von 0,01 bis 7,0 Gewichtsteilen bezogen auf 100 Gewichtsteile des Trägers vorhanden ist.

5. Katalysator gemäß Ansprüchen 1 bis 4, wobei die Metallkomponente aus Platin und Zinn besteht.

6. Katalysator gemäß Ansprüche 1 bis 2 oder Anspruch 4, wobei die Metallkomponente aus Platin und Blei besteht.

7. Verfahren zur Herstellung eines Katalysators gemäß einem der Ansprüche 1 bis 6, umfassend die Schritte:
(1) Formen eines Trägers, der umfasst
(a) 10 bis 80 Gew.-% von mindestens einem von
(i) Mordenit und
(ii) Zeolith vom beta-Typ;
das Mordenit und das Zeolith vom beta-Typ haben ein Molverhältnis von Silica zu Alumina von 10 bis 200;
(b) 15 bis 70 Gew.-% Zeolith vom ZSM-5-Typ mit einem Molverhältnis von Silica zu Alumina von 30 bis 500; und
(c) 5 bis 90 Gew.-% mindestens eines organischen Bindemittels ausgewählt aus der Gruppe bestehend aus Gamma-Alumina, Silica, Silica-Alumina, Bentonit, Kaolin, Clinoptilolith und Montmorillonit;
(2) Aufbringen von 0,01 bis 10,0 Gewichtsteilen Zinn oder 0,01 bis 7,0 Gewichtsteilen Blei in 100 Gewichtsteilen Träger; und
(3) Aufbringen von 0,001 bis 0,5 Gewichtsteilen Platin auf diesen zinn- oder bleigeträgerten Träger bezogen auf 100 Gewichtsteile des Trägers, um den Katalysator zu erhalten.

8. Verfahren zur Herstellung eines Katalysators gemäß einem der Ansprüche 1 bis 6, umfassend die Schritte:
(1) Vermischen von
(a) 10 bis 80 Gew.-% von mindestens einem von
(i) Mordenit und
(ii) Zeolith vom beta-Typ;
das Mordenit und das Zeolith vom beta-Typ haben ein Molverhältnis von
Silica zu Alumina von 10 bis 200;
(b) 15 bis 70 Gew.-% Zeolith vom ZSM-5-Typ mit einem Molverhältnis von Silica zu Alumina von 30 bis 500;
(c) 5 bis 90 Gew.-% von mindestens einem organischen Bindemittel ausgewählt aus der Gruppe bestehend aus Gamma-Alumina, Silica, Silica-Alumina, Bentonit, Kaolin, Clinoptilolith und Montmorillonit;
(d) 0,01 bis 10 Gewichtsteilen Zinn oder 0,01 bis 7,0 Gewichtsteilen Blei in 100 Gewichtsteilen Träger; und
(e) 0,001 bis 0,5 Gewichtsteilen Platin in 100 Gewichtsteilen des Trägers;
(2) Formen der Mischung, um einen Katalysator zu erhalten.

9. Verfahren zu Herstellung eines Katalysators gemäß einem der Ansprüche 1 bis 6, umfassend die Schritte:
(1) Aufbringen von Platin auf mindestens einem von
(i) Mordenit und
(ii) Zeolith vom beta-Typ;
das Mordenit und das Zeolith vom beta-Typ haben ein Molverhältnis von Silica zu Alumina von 10 bis 200;
durch Imprägnierung oder durch lonenaustausch;
(2) Formen des Platins aufgeträgert auf mindestens einem von (i) Mordenit und (ii) Zeolith vom beta-Typ mit Zeolith vom ZSM-5 Typ mit einem Molverhältnis von Silica zu Alumina von 30 bis 500 und mindestens einem organischen Bindemittel ausgewählt aus der Gruppe bestehend aus Gamma-Alumina, Silica, Silica-Alumina, Bentonit, Kaolin, Clinoptilolith und Montmorillonit in eine bestimmte Form;
wobei
das mindestens eine von
(i) Mordenit und
(ii) Zeolith vom beta-Typ;
das Zeolith vom ZSM-5-Typ und der anorganische Binder
vorhanden sind in einer Menge von 10 bis 80 Gew.-%, 15 bis 70 Gew.-% bzw. 5 bis 90 Gew.-% und das Platin in einer Menge von 0,001 bis 0,5 Gewichtsteilen bezogen auf 100 Gewichtsteile des Trägers bestehend aus Zeolithen und Bindemitteln vorhanden ist und
(3) Aufbringen von Zinn oder Blei auf diese geformte Form in einer Menge von 0,01 bis 10,0 Gewichtsteilen bzw. 0,01 bis 7,0 Gewichtsteilen, bezogen auf 100 Gewichtsteile des Trägers, um den Katalysator zu erhalten.

10. Ein Verfahren zur Herstellung eines Katalysators gemäß einem der Ansprüche 1 bis 6, umfassend die Schritte:
(1) Aufbringen von Platin auf eine Mischung von
(a) mindestens einem von
(i) Mordenit und
(ii) Zeolith vom beta-Typ;
das Mordenit und das Zeolith vom beta-Typ haben ein Molverhältnis von Silica zu Alumina von 10 bis 200; und
(b) Zeolith vom ZSM-5-Typ mit einem Molverhältnis von Silica zu Alumina von 30 bis 500;
durch Imprägnierung oder Ionenaustausch;
(2) Formen des Platin geträgerten Zeoliths zusammen mit mindestens einem anorganischen Bindemittel ausgewählt aus der Gruppe bestehend aus Gamma-Alumina, Silica, Silica-Alumina, Bentonit, Kaolin, Clinoptilolith und Montmorillonit in eine bestimmte Form, wobei das Mordenit und/oder Zeolith vom beta-Typ, das Zeolith vom ZSM-5 Typ, und der Binder vorhanden sind in einer Menge von 10 bis 80 Gew.-%, 15 bis 70 Gew.-% bzw. 5 bis 90 Gew.-%, und das Platin ist in einer Menge von 0,001 bis 5 Gewichtsteilen bezogen auf 100 Gewichtsteile Träger, bestehend aus Zeolithen und dem Bindemittel, vorhanden; und
(3) Aufbringen von Zinn oder Blei in diese geformte Form mit einer Menge von 0,01 bis 10 Gewichtsteilen bzw. 0,01 bis 7,0 Gewichtsteilen bezogen auf 100 Gewichtsteile Träger, um diesen Katalysator zu erhalten.

11. Verfahren zur Herstellung eines Katalysators gemäß einem der Ansprüche 1 bis 6 umfassend die Schritte:
(1) Aufbringen von Zinn oder Blei auf mindestens einem von
(i) Mordenit und
(ii) Zeolith vom beta-Typ;
das Mordenit und das Zeolith vom beta-Typ haben ein
Molverhältnis von Silica zu Alumina von 10 bis 200; durch
Imprägnierung oder lonenaustausch;
(2) Formen des mindestens einen
(i) zinn- oder bleigeträgerten Mordenits; und
(ii) zinn- oder bleigeträgerten Zeoliths vom beta-Typ
mit ZSM-5 Zeolith mit einem Molverhältnis von Silica zu Alumina von 300 bis 500; und mindestens einem anorganischen Bindemittel ausgewählt aus der Gruppe bestehend aus Gamma-Alumina, Silica, Silica-Alumina, Bentonit, Kaolin, Clinoptilolith und Montmorillonit in eine bestimmte Form;
wobei der mindestens eine von
(i) Mordenit, und
(ii) Zeolith vom beta-Typ;
das Zeolith vom ZSM-5 Typ und der anorganische Binder in einer Menge vorhanden sind von 10 bis 80 Gew.-%, 15 bis 70 Gew.-% bzw. 5 bis 90 Gew.-% und das Zinn in einer Menge vorhanden ist von 0,01 bis 10 Gewichtsteilen oder das Blei ist in einer Menge von 0,01 bis 7,0 Gewichtsteilen vorhanden, bezogen auf 100 Gewichtsteile des Trägers;
(3) Aufbringen von 0,001 bis 0,5 Gewichtsteilen Platin bezogen auf 100 Gewichtsteile Träger in die ausgebildete Form, um den Katalysator zu erhalten.

12. Verfahren zu Herstellung eines Katalysators gemäß einem der Ansprüche 1 bis 6 umfassend die Schritte:
(1) Aufbringen von Zinn oder Blei auf eine Mischung von
(a) einem von
(i) Mordenit und
(ii) Zeolith vom beta-Typ;
das Mordenit und das Zeolith vom beta-Typ haben ein Molverhältnis von Silica zu Alumina von 10 bis 200; und
(b) ZSM-5 Zeolith mit einem Molverhältnis von Silica zu Alumina von 30 bis 500 durch Imprägnierung oder lonenaustausch;
(2) Formen des Zinn- oder Blei geträgerten Zeoliths zusammen mit mindestens einem anorganischen Bindemittel ausgewählt aus der Gruppe bestehend aus Gamma-Alumina, Silica, Silica-Alumina, Bentonit, Kaolin, Clinoptilolith und Montmorillonit in eine bestimmte Form, wobei das Mordenit und/oder Zeolith vom beta-Typ, das ZSM-5 Zeolith und das Bindemittel in einer Menge vorhanden sind von 10 bis 80 Gew.-%, 15 bis 70 Gew.-% bzw. 5 bis 90 Gew.-% und das Zinn in einer Menge vorhanden ist von 0,01 bis 10 Gewichtsteilen oder das Blei in einer Menge vorhanden ist von 0,01 bis 7 Gewichtsteilen bezogen auf 100 Gewichtsteile der Zeolithe und des Bindemittels;
(3) Aufbringen von Platin in diese geformte Form mit einer Menge von 0,001 bis 0,5 Gewichtsteilen bezogen auf 100 Gewichtsteile Träger, um den Katalysator zu erhalten.

13. Verfahren zur Herstellung von Mischxylolen aus aromatischen Kohlenwasserstoffen durch Dealkylierung, Disproportionierung oder Transalkylierung unter Verwendung des Katalysators gemäß einem der Ansprüche 1 bis 6.

## Revendications

1. Catalyseur comprenant :
(1) un véhicule comprenant :
(a) 10 à 80 % en poids d'au moins une parmi
(i) la mordénite et
(ii) la zéolite de type bêta,
ladite mordénite et ladite zéolite de type bêta ayant un rapport molaire de la silice à l'alumine de 10 à 200,
(b) 15 à 70 % en poids d'une zéolite de type ZSM-5 ayant un rapport molaire de la silice à l'alumine de 30 à 500 ; et
(c) 5 à 90 % en poids d'au moins un liant inorganique choisi parmi le groupe constitué d'alumine-gamma, silice, silice-alumine, bentonite, kaolin, clinoptilolite et montmorillonite ; et
(2) un composant métallique, supporté sur ledit véhicule, ledit composant métallique étant constitué de :
(a) platine et
(b) soit de l'étain soit du plomb.

2. Catalyseur selon la revendication 1, dans lequel le platine est présent dans le véhicule en une quantité de 0,001 à 0,5 partie en poids sur la base de 100 parties en poids du véhicule.

3. Catalyseur selon les revendications 1 à 2, dans lequel l'étain est présent dans le véhicule en une quantité de 0,01 à 10,0 parties en poids sur la base de 100 parties en poids du véhicule.

4. Catalyseur selon les revendications 1 à 3, dans lequel le plomb est présent dans le véhicule en une quantité de 0,01 à 7,0 parties en poids sur la base de 100 parties en poids du véhicule.

5. Catalyseur selon les revendications 1 à 4, dans lequel le composant métallique est constitué de platine et d'étain.

6. Catalyseur selon les revendications 1 à 2 ou la revendication 4, dans lequel le composant métallique est constitué de platine et de plomb.

7. Procédé pour la préparation d'un catalyseur selon l'une quelconque des revendications 1 à 6, comprenant les étapes consistant à :
(1) former un véhicule qui comprend :
(a) 10 à 80 % en poids d'au moins une parmi
(i) la mordénite et
(ii) une zéolite de type bêta ;
ladite mordénite et ladite zéolite de type bêta ayant un rapport molaire de la silice à l'alumine de 10 à 200 ;
(b) 15 à 70 % en poids de zéolite de type ZSM-5 ayant un rapport molaire de la silice à l'alumine de 30 à 500 ; et
(c) 5 à 90 % en poids d'au moins un liant inorganique choisi parmi le groupe constitué d'alumine-gamma, silice, silice-alumine, bentonite, kaolin, clinoptilolite et montmorillonite.
(2) supporter 0,01 à 1,0 parties en poids d'étain ou 0,01 à 7,0 parties en poids de plomb dans 100 parties en poids dudit véhicule ; et
(3) supporter 0,001 à 0,5 parties en poids de platine sur ledit véhicule supportant l'étain ou le plomb, sur la base de 100 parties en poids dudit véhicule, pour donner ledit catalyseur.

8. Procédé pour la préparation d'un catalyseur selon l'une quelconque des revendications 1 à 6, comprenant les étapes consistant à :
(1) mélanger
(a) 10 à 80 % en poids d'au moins une parmi :
(i) la mordénite et
(ii) une zéolite de type bêta ;
ladite mordénite et ladite zéolite de type bêta ayant un rapport molaire de la silice à l'alumine de 10 à 200 ;
(b) 15 à 70 % en poids de zéolite de type ZSM-5 ayant un rapport molaire de la silice à l'alumine de 30 à 500;
(c) 5 à 90 % en poids d'au moins un liant inorganique choisi parmi le groupe constitué d'alumine-gamma, silice, silice-alumine, bentonite, kaolin, clinoptilolite et montmorillonite.
(d) 0,01 à 10,0 parties en poids d'étain ou 0,01 à 7,0 parties en poids de plomb dans 100 parties en poids du véhicule ; et
(e) 0,001 à 0,5 parties en poids de platine dans 100 parties en poids du véhicule; et
(2) mouler le mélange pour donner ledit catalyseur.

9. Procédé pour la préparation d'un catalyseur selon l'une quelconque des revendications 1 à 6, comprenant les étapes consistant à :
(1) supporter le platine sur au moins une parmi :
(i) la mordénite et
(ii) une zéolite de type bêta ;
ladite mordénite et ladite zéolite de type bêta ayant un rapport molaire de la silice à l'alumine de 10 à 200 ;
par imprégnation ou échange ionique ;
(2) mouler ledit platine, supporté sur au moins une parmi (i) la mordénite et (ii) une zéolite de type bêta, avec une zéolite de type ZSM-5 ayant un rapport molaire de la silice à l'alumine de 30 à 500, et au moins un liant inorganique choisi parmi le groupe constitué d'alumine-gamma, silice, silice-alumine, bentonite, kaolin, clinoptilolite et montmorillonite sous une certaine forme ;
dans lequel :
au moins une parmi :
(i) la mordénite et
(ii) une zéolite de type bêta ;
la zéolite de type ZSM-5 et
le liant inorganique, sont présents en une quantité de 10 à 80 % en poids, 15 à 70 % en poids et 5 à 90 % en poids, respectivement et le platine est présent en une quantité de 0,001 à 0,5 partie en poids sur la base de 100 parties en poids du véhicule constitué de zéolites et de liant et
(3) supporter l'étain ou le plomb sous ladite forme moulée en une quantité de 0,01 à 10,0 parties en poids ou 0,01 à 7,0 parties en poids, respectivement, sur la base de 100 parties en poids dudit véhicule, pour donner ledit catalyseur.

10. Procédé pour la préparation d'un catalyseur selon l'une quelconque des revendications 1 à 6, comprenant les étapes consistant à :
(1) supporter du platine sur un mélange de :
(a) au moins une parmi
(i) la mordénite et
(ii) une zéolite de type bêta ;
ladite mordénite et ladite zéolite de type bêta ayant un rapport molaire de la silice à l'alumine de 10 à 200 ; et
(b) zéolite ZSM-5 dans un rapport molaire de la silice à l'alumine de 30 à 500 ;
par imprégnation ou échange ionique ;
(2) mouler la zéolite supportant le platine conjointement avec au moins un liant inorganique choisi parmi le groupe constitué d'alumine-gamma, silice, silice-alumine, bentonite, kaolin, clinoptilolite et montmorillonite, sous une certaine forme dans laquelle la mordénite et/ou la zéolite de type bêta, la zéolite ZSM-5 et le liant sont présents en une quantité de 10 à 80 % en poids, 15 à 70 % et 5 à 90 % en poids, respectivement, et le platine est présent en une quantité de 0,001 à 5 parties en poids sur la base de 100 parties en poids du véhicule constitué des zéolites et du liant ; et
(3) supporter l'étain ou le plomb sous ladite forme moulée en une quantité de 0,01 à 10,0 parties en poids ou 0,01 à 7,0 parties en poids, respectivement, sur la base de 100 parties en poids du véhicule, pour donner ledit catalyseur.

11. Procédé pour la préparation d'un catalyseur selon l'une quelconque des revendications 1 à 6, comprenant l'étape consistant à
(1) supporter l'étain ou le plomb sur au moins une parmi
(i) la mordénite et
(ii) une zéolite de type bêta ;
ladite mordénite et ladite zéolite de type bêta ayant un rapport molaire de la silice à l'alumine de 10 à 200 ;
par imprégnation ou échange ionique
(2) mouler au moins une parmi
(i) la mordénite supportant l'étain ou le plomb ; et
(ii) la zéolite de type bêta supportant l'étain ou le plomb ;
avec
la zéolite ZSM-5 ayant un rapport molaire de la silice à l'alumine de 30 à 500 ;
et
au moins un liant inorganique choisi parmi le groupe constitué d'alumine-gamma, silice, silice-alumine, bentonite, kaolin, clinoptilolite et montmorillonite, sous une certaine forme ;
dans lequel ;
la au moins une parmi
(i) la mordénite ; et
(ii) une zéolite de type bêta ;
la zéolite de type ZSM-5 et
le liant inorganique
sont présents en une quantité de 10 à 80 % en poids, 15 à 70 % en poids et 5 à 90 % en poids, respectivement et ledit étain est présent en une quantité de 0,01 à 10 parties en poids ou ledit plomb est présent en une quantité de 0,01 à 7,0 parties en poids sur la base de 100 parties en poids dudit véhicule ; et
(3) supporter 0,001 à 0,5 partie en poids de platine, sur la base de 100 parties en poids du véhicule, sous ladite forme moulée, pour donner ledit catalyseur.

12. Procédé pour la préparation d'un catalyseur selon l'une quelconque des revendications 1 à 6, comprenant les étape consistant à :
(1) supporter l'étain ou le plomb sur un mélange de
(a) un parmi
(i) la mordénite et
(ii) une zéolite de type bêta ;
ladite mordénite et ladite zéolite de type bêta ayant un rapport molaire de la silice à l'alumine de 10 à 200 ; et
(b) une zéolite ZSM-5 ayant un rapport molaire de la silice à l'alumine de 30 à 500,
par imprégnation ou échange ionique ;
(2) mouler la zéolite supportant l'étain ou le plomb conjointement avec au moins un liant inorganique choisi parmi le groupe constitué d'alumine-gamma, silice, silice-alumine, bentonite, kaolin, clinoptilolite et montmorillonite, sous une certaine forme dans laquelle la mordénite et/ou la zéolite de type bêta, la zéolite ZSM-5 et le liant sont présents en une quantité de 10 à 80 % en poids, 15 à 70 % en poids et 5 à 90 % en poids, respectivement, et l'étain est présent en une quantité de 0,01 à 10 parties en poids ou le plomb est présent en une quantité de 0,01 à 7 parties en poids sur la base de 100 parties en poids dudit zéolites et liant ; et
(3) supporter le platine sous ladite forme moulée en une quantité de 0,001 à 0,5 partie en poids, sur la base de 100 parties en poids du véhicule, pour donner ledit catalyseur.

13. Procédé de production de xylènes mélangés à partir d'hydrocarbures aromatiques par désalkylation, dismutation ou transalkylation en utilisant le catalyseur selon l'une quelconque des revendications 1 à 6.
